# EUROPEAN PATENT APPLICATION

(11) **EP 2 689 838 A1**
(43) Date of publication of application: **29.01.2014**
(21) Application number: 12178025.8
(22) Date of filing: 26.07.2012
(51) Int. Cl.: B01J 19/00, C08F 2/00, B08B 7/02

(54) **Method for cleaning a reactor**

(71) Applicant: Saudi Basic Industries Corporation, Riyadh 11422 (SA); Linde AG, 80331 Munich (DE)
(72) Inventor: Dr. Alqahtani, Abdullah, Riyadh 11551 (SA); Dr. Lliyas, Abuljelil, Riyadh 11333 (SA); Azam, Shahid, Bihar (IN); Meiswinkel, Andreas, 81479 Munich (DE); Wöhl, Anina, 81379 Munich (DE); Müller, Wolfgang, 81245 Munich (DE); Haff, Marco, 80337 Munich (DE); Hoffmann, Karl-Heinz, 82110 Germering (DE); Zander, Hans-Jörg, 81479 Munich (DE); Bölt, Heinz, 82515 Wolfratshausen (DE)
(74) Representative: Scholz, Volker

(57) **Abstract**

The present invention relates to a method for cleaning a reactor which is suitable for carrying out a chemical process, the reactor having solid deposits as product of the chemical process on reactor walls and containing processing medium and inert solid abrasive particles, wherein the inert solid abrasive particles are stirred together with the processing medium so that the inert solid abrasive particles impact on the reactor walls.

## Description

The present invention relates to a method for cleaning a reactor suitable for carrying out a chemical process.

Many chemical processes are known which are carried out in a reactor having a tendency of fouling by deposition of solids.

Reactor fouling is especially a problem related to polymer technologies, i.e. oligomerization and polymerization reactions, for example oligomerization of ethylene, which always results in the production of some amounts of solid polymeric product which forms deposits on the reactor walls and any equipment in contact therewith.

As a consequence of "fouling", the reactor has to be cleaned periodically, and, preferably, a spare reactor is required to maintain production.

Nowadays, mechanical cleaning, cleaning by hydro-jetting or by purging with a suitable cleaning agent is known.

EP 1 752 212 A1 discloses a method for cleaning a reactor having a tendency of fouling by deposition of solids, wherein a hot solvent, in which the solids are soluble and which has a temperature of at least about 75°C, is applied to the reactor, the solids being substantially dissolved and the dissolved solids being discharged from the reactor, wherein the method is carried out without opening the reactor to atmosphere.

EP 1 777 210 A1 discloses a method for oligomerization of ethylene and/or alpha-olefins utilizing reactor equipment and other equipment wherein the reactor equipment is flushed with a reaction product fraction obtained by said our respective previous method prior to and/or after that oligomerization, wherein the reaction product fraction is a fraction of inert alpha-olefins containing C₁₂ - C₁₈ product.

It is a disadvantage of the methods for cleaning a reactor as described above that it is always necessary to shut down the reactor operation in order to effect cleaning.

In the prior art, there is usually a phase of time necessary after cleaning in order to put the reactor again under process conditions, in which phase no or only restricted production can take place. Here, inertizing, drying and filling with processing medium can be mentioned. Further, prior to opening the reactor for mechanical cleaning, the reactor has regularly first to be inertized and then to be vented, in order to effect a safe cleaning without jeopardizing of men and environment. The cleaning measures as such are related to significant efforts (machines, material, personnel). All these cleaning measures result in production losses or, in order to guarantee a constant production, require the provision of spare reactors.

It is thus the object of the present invention to provide a method for cleaning a reactor suitable for carrying out a chemical process, which overcomes the disadvantages of the prior art, especially to provide a method which requires no or only minimum downtime of the reactor operation which can be carried out without opening and/or emptying the reactor.

This object is achieved by a method for cleaning a reactor which is suitable for carrying out a chemical process, the reactor having solid deposits as product of the chemical process on reactor walls and containing processing medium and inert solid abrasive particles, wherein the inert solid abrasive particles are stirred together with the processing medium so that the inert solid abrasive particles impact on the reactor walls.

Preferably, the method is simultaneously carried out with the chemical process in the reactor.

In one preferred embodiment the processing medium is substantially liquid or substantially gaseous-liquid.

The processing medium may contain homogenous or heterogeneous catalyst.

Moreover preferably, the chemical process is an oligomerization or polymerization of olefins, preferably the oligomerization ethylene.

In a most preferred embodiment of the invention, the method for cleaning the reactor is simultaneously carried out with the oligomerization of ethylene in the reactor. Such oligomerization may comprise the steps of:
a. feeding ethylene into the oligomerization reactor,
b. oligomerizing the ethylene in the reactor,
c. removing a reactor outlet stream (processing medium) comprising linear alpha-olefins from the reactor via a reactor outlet piping system,
d. optionally transferring the reactor outlet stream to a catalyst deactivation and removal step, and
e. optionally deactivating and removing the catalyst from the reactor outlet stream.

In such an oligomerization reaction, the inert solid abrasive particles can be already present in the reactor when initiating the oligomerization, and can be either removed together with the processing medium or can be kept in the reactor as outlined in preferred embodiments below.

In a preferred embodiment, the inert solid abrasive particles are selected from the group consisting of steel, scrap metal, glass, ceramic, carbide, quartz, SiO₂ and high density polyethylene (HDPE).

Preferably, the particles have a diameter of 0.1 mm - 10 cm, preferably 5 mm - 5 cm.

In a most preferred embodiment the inert solid abrasive particles have a density which does differ in the density of the processing medium by not more than 10 %, preferably by not more than 5 %. A suitable density of the inert solid abrasive particles can be ensured with accurate instruments, for example density meter, etc.

In one embodiment the inert solid abrasive particles are hollow particles.

It is preferred, the inert solid abrasive particles are mechanically stable.

Furthermore, the reactor may be a stirred vessel, a bubble column reactor or a loop reactor.

In a preferred embodiment, preferably after stirring, the particles and the processing medium are transferred to a separation unit and separated.

Most preferably, the separation unit is a mesh, a filter or a cyclone.

It is preferred that the separated inert solid abrasive particles are regenerated and/or re-introduced into the reactor. The inert solid abrasive particles can be directly recycled into the reactor or, in a first step, mixed with any inlet fluid (s).

Finally, it is preferred that separation is such that the processing medium is removed from the reactor and the inert solid abrasive particles remain within the reactor. If desired, the particles remaining in the reactor can be regenerated using a suitable solvent during the reactor shut down.

It was surprisingly found that according to the inventive method cleaning of a reactor can be effected without the necessity of opening and/or emptying the reactor, so that emptying and re-implementing times of the reactor can be saved.

In a particularly preferred embodiment of the inventive method, the cleaning operation can be simultaneously carried out with the chemical process conducted in the reactor, for example oligomerization or polymerization, i.e. during operation of the reactor. Thus, downtimes can be significantly reduced and production times extended, as times for cleaning, including start-up and shut down times, can be saved in which no operation is possible. Further, cleaning costs and separating cleaning and flushing systems can be saved. Where appropriate, one or more spare reactors can be set aside, which otherwise would be necessary for maintaining the production capacity, if one reactor is cleaned. Thus, both investment as well as operational costs can be saved.

The term "reactor" in the present invention is to be understood to not only comprise the reactor itself, but shall also include any equipment connected with the reactor which is prone to show solid deposits which have to be removed for safe operation.

The shape of the inert solid abrasive particles is not of particular relevance. The shape can be for example spherical.

The term "processing medium" as used in the present application is to be understood as to comprise educts, products, solvents and, if applicable, homogeneous or heterogeneous catalysts. The processing medium can contain aqueous, organic or multiphase solutions or mixtures, for example liquid/gaseous/solid (for example heterogeneous catalyst) mixtures. The solid abrasive particles have to be inert to the processing medium. The material of these particles has to be sufficiently hard and/or mechanically stable. Mechanically stable means in the context of the present invention that the particles do not alter during cleaning operation, for example the particles do not break or do not show abrasion themselves.

For the inventive method it is essential that there is at least a sufficient mixing of processing medium and inert solid abrasive particles so that the particles may impact on the reactor walls to allow continuous removal of the deposits. The removed deposits can suspend in the processing medium and can be discharged off the reactor.

The method of the present invention can be conducted out principally under all process conditions, both at deep or high temperature, and within all pressure ranges, preferably within the range of 1 bar to 100 bar.

In a preferred embodiment, the inert solid abrasive particles can be separated from the processing medium after stirring. In this regard, it is, as a first alternative, possible to completely discharge the processing medium containing the inert solid abrasive particles from the reactor and separating the particles, for example, by filtration or using a cyclone. In this alternative, the inert solid abrasive particles have to be preferably re-introduced into the reactor in order to allow constant concentration/distribution of the particles within the reactor. It may be necessary to regenerate the particles, as there may be also respective deposits thereon. The regeneration strongly depends from the material of the inert solid abrasive particles and the type of deposits. Regeneration could be, for example, obtained by washing with a suitable solvent, firing, etc.

According to a second alternative, it is also possible to separate processing medium and inert solid abrasive particles by having a respective separation unit, such as a mesh or a filler, at the reactor outlet so that the particles remain in the reactor and only the processing medium can be discharged. Using this alternative the regeneration and recycling steps are not necessary.

### Comparative example

A process for the oligomerization of ethylene is conducted in order to prepare linear alpha-olefins. This process is carried out in a bubble column reactor by introducing ethylene into the reactor containing solvent and catalyst. The process is a continuous process. After several days of processing, the oliogmerization is shut down and the reactor inspected. The reactor walls show significant deposits of polymeric materials which have to be removed.

### Inventive example

The oligomerization of ethylene is conducted out as described above with regard to the comparative example, however with the addition of inert solid abrasive particles (quartz) in the bubble column reactor. The particles have a spherical shape and a diameter of about 1 cm. The concentration of the particles in the reactor was adjusted to be present in an amount of about 1.000 particles per cubic meter. Discharge of these particles is prohibited by having respective meshes of suitable sizes at the reactor outlets. Mixing of the processing medium with the inert solid abrasive particles is sufficient so that the particles may impact on the reactor walls. The oligomerization is conducted also for several days and is then shut down and the reactor opened for inspection. Only neglectable deposits can be found.

The features disclosed in the foregoing description and the claims may, both separately and in any combination thereof, be material for realizing the invention in diverse forms thereof.

## Claims

1. Method for cleaning a reactor which is suitable for carrying out a chemical process, the reactor having solid deposits as product of the chemical process on reactor walls and containing processing medium and inert solid abrasive particles, wherein the inert solid abrasive particles are stirred together with the processing medium so that the inert solid abrasive particles impact on the reactor walls.

2. Method according to claim 1, which is simultaneously carried out with the chemical process in the reactor.

3. Method according to claim 1 or 2, wherein the processing medium is substantially liquid or substantially gaseous-liquid.

4. Method according to any of the preceding claims, wherein the processing medium contains homogenous or heterogeneous catalyst.

5. Method according to any of the preceding claims, wherein the chemical process is an oligomerization or polymerization of olefins, preferably the oligomerization ethylene.

6. Method according to any of the preceding claims, wherein the inert solid abrasive particles are selected from the group consisting of steel, scrap metal, glass, ceramic, carbide, quartz, SiO₂ and high density polyethylene (HDPE).

7. Method according to any of the preceding claims, wherein the particles have a diameter of 0.1 mm - 10 cm, preferably 5 mm - 5 cm.

8. Method according to any of the preceding claims, wherein the inert solid abrasive particles have a density which does differ in the density of the processing medium by not more than 10 %, preferably by not more than 5 %.

9. Method according to any of the preceding claims, wherein the inert solid abrasive particles are hollow particles.

10. Method according to any of the preceding claims, wherein the inert solid abrasive particles are mechanically stable.

11. Method according to any of the preceding claims, wherein the reactor is a stirred vessel, a bubble column reactor or a loop reactor.

12. Method according to any of the preceding claims, wherein, preferably after stirring, the particles and the processing medium are transferred to a separation unit and separated.

13. Method according to claim 12, wherein the separation unit is a mesh, a filter or a cyclone.

14. Method according to claim 12 or 13, wherein the separated inert solid abrasive particles are regenerated and/or re-introduced into the reactor.

15. Method according to claim 12 or 13, wherein separation is such that the processing medium is removed from the reactor and the inert solid abrasive particles remain within the reactor.
